# EUROPEAN PATENT APPLICATION

(11) **EP 1 889 610 A2**
(43) Date of publication of application: **20.02.2008**
(21) Application number: 07253251.8
(22) Date of filing: 17.08.2007
(51) Int. Cl.: A61K 9/20, A61K 31/277

(54) **Bicalutamide compositions**

(30) Priority: 17.08.2006 IN CH14492006; 02.10.2006 US 827727 P
(71) Applicant: Dr. Reddy's Laboratories Ltd., Hyderabad 500 016, Andhra Pradesh (IN)
(72) Inventor: Gawande, Rahul Sudhaker, Nagpur 440 015 Maharashtra (IN); Basety, Srikanth, Hyderabad 500 001 Andhra Pradesh (IN); Kodipyaka, Ravinder, Hyderabad 500 072 Andhra Pradesh (IN); Bhushan, Indu, Hyderabad 500 072 Andhra Pradesh (IN); Mohan, Maliatur Sivaraman, Hyderabad 500 072 Andhra Pradesh (IN)
(74) Representative: Bates, Philip Ian

(57) **Abstract**

Pharmaceutical compositions comprising bicalutamide or its pharmaceutically acceptable salts, solvates, single isomers, enantiomers or mixtures thereof, processes for preparing the same and methods of use and treatment.

## Description

### INTRODUCTION TO THE INVENTION

The present invention relates to pharmaceutical compositions comprising bicalutamide or its pharmaceutically acceptable salts, solvates, single isomers, enantiomers or mixtures thereof, processes for preparing the same and methods of use and treatment. More particularly, the present invention relates to stable pharmaceutical compositions providing enhanced solubility parameters of bicalutamide.

Bicalutamide has a chemical name (R,S)-N-(4-cyano-3-(4-fluorophenylsulfonyl)-2-hydroxy-2-methyl-3-(trifluoro-methyl)propionanilide), with the structural Formula I. It is nonsteroidal anti-androgenic agent. The drug is used in combination with surgical castration or leutinizing hormone-releasing hormone (LHRH) analog therapy. It has affinity for androgen receptors, but not for progestogen, estrogen, or glucocorticoid receptors. Consequently, it blocks the action of androgens of adrenal and testicular origin, which stimulate the growth of normal and malignant prostatic tissue. Solubility of bicalutamide is 5 mg/1000 mL in water at 37°C. Bicalutamide is commercially available under the trademark CASODEX^{®} as 50 mg tablets for oral administration, manufactured by AstraZeneca.

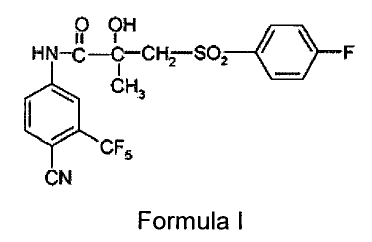

The rate of dissolution of a poorly-soluble drug is a rate-limiting factor in its absorption by the body. Different approaches to enhance dissolution rate of poorly soluble drug comprise micronisation, complexation with cyclodextrins, use of solubilizers and hydrophilic surfactants, solid dispersions, and the like. A reduction in the particle size increases the dissolution rate of such compounds through an increase in the surface area of the solid phase that comes in contact with the liquid medium, thereby resulting in an enhanced bioavailability of the compositions containing such compounds. However, handling of very fine particles is a problem because of static charge generation during milling, causing it to agglomerate which then poses problems in flowability, compression and uniform particle size distribution. Furthermore, milling of an active pharmaceutical ingredient is a dusty operation, which is hazardous since bicalutamide is potent and undue exposure during milling of the drug may cause serious side effects of the personnel involved in the operation. It is generally not possible to predict the exact particle size and distribution required for any particular drug substance to achieve a specific dissolution profile or a specific in vivo behavior, as different drugs show differing dissolution characteristics with a reduction in the particle size. The problem is further complicated by the fact that the same compound may exist in more than one crystalline form or a crystalline form, each of which could have a completely different dissolution profile. Problems as stated above can be overcome by the use of stable pharmaceutical compositions comprising amorphous form of such poorly soluble drugs like bicalutamide.

U.S. Patent Application Publication No. 2006/0058381 describes a pharmaceutical formulation comprising bicalutamide, wherein greater than 50% of bicalutamide is in the form of the R-enantiomer.

U.S. Patent Application Publication Nos. 2004/0138299 and 2004/0067257 describe formulations of bicalutamide with polyvinylpyrrolidone, and with an enteric polymer having a pKₐ from 3 to 6, respectively.

U.S. Patent No. 6,861,557 and U.S. Patent Application Publication No. 2005/0008691 respectively describe micronized racemic bicalutamide and a granulate comprising micronized bicalutamide.

United States Patent Application Publication No. 2006/0286162 describes an adsorbate, comprising an adsorbent and bicalutamide adsorbed on said adsorbent. The process comprises the steps of providing a suspension of said adsorbent in a solution of bicalutamide and recovering said adsorbate from said suspension.

European Patent Application No. 1027886 A describes a composition comprising a solid dispersion comprising a low-solubility drug and at least one polymer. At least a major portion of the drug in the dispersion is amorphous. The polymer has a glass transition temperature of at least 100°C measured at a relative humidity of fifty percent.

Development of stable pharmaceutical compositions providing enhanced solubility parameters of bicalutamide as described in the context of the present invention would be a significant improvement in the field of pharmaceutical technology.

### SUMMARY OF THE INVENTION

The present invention relates to pharmaceutical compositions comprising bicalutamide or its pharmaceutically acceptable salts, solvates, single isomers, enantiomers or mixtures thereof, processes for preparing the same and methods of use and treatment.

More particularly, the present invention relates to pharmaceutical compositions providing enhanced solubility parameters of bicalutamide prepared using one or more hydrophilic excipients, which results in a desired dissolution profile.

More particularly, the present invention relates to pharmaceutical compositions providing enhanced solubility parameters of bicalutamide prepared using hydrophilic excipient(s), other than polymers that have pH-dependent solubility or with a pKₐ between about 3 and about 6, which result in a desired dissolution profile.

More particularly, the present invention relates to pharmaceutical compositions providing enhanced solubility parameters of bicalutamide prepared using hydrophilic excipient(s), other than polyvinylpyrollidones, which result in a desired dissolution profile.

Further, the present invention relates to stable pharmaceutical compositions providing enhanced solubility parameters of bicalutamide, which do not show changes in their XRD patterns upon storage.

### BRIEF DESCRIPTION OF THE DRAWINGS

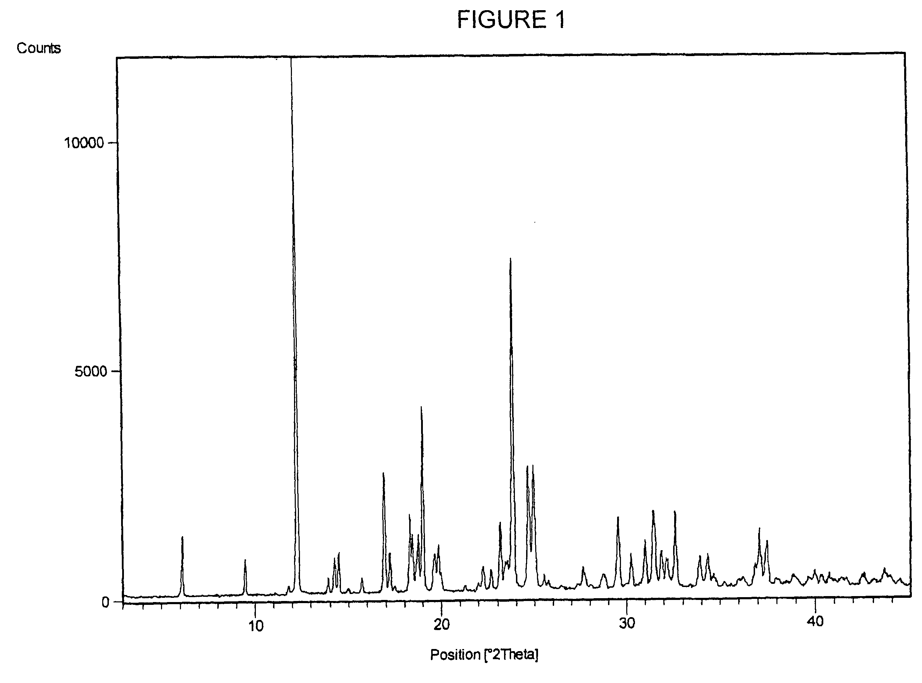

Figure 1 is a X-ray powder diffraction ("XRD") pattern of pure bicalutamide.
Figure 2 is a XRD pattern of bicalutamide tablets 50 mg prepared in Example 6.
Figure 3 is a XRD pattern of bicalutamide tablets 50 mg prepared in Example 6, after storage for 3 months at 40 °C and 75% relative humidity ("RH").
Figure 4 is a XRD pattern for a placebo tablet, prepared according to Example 6 but without any bicalutamide.

### DETAILED DESCRIPTION OF THE INVENTION

The present invention relates to pharmaceutical compositions comprising bicalutamide or its pharmaceutically acceptable salts, solvates, single isomers, enantiomers or mixtures thereof, processes for preparing the same and methods of use and treatment.

More particularly, the present invention relates to pharmaceutical compositions providing enhanced solubility parameters of bicalutamide prepared using one or more hydrophilic excipient(s), which result in a desired dissolution profile.

More particularly, the present invention relates to pharmaceutical compositions providing enhanced solubility parameters of bicalutamide prepared using one or more hydrophilic excipient(s), other than "enteric" polymers that have pH-dependent solubility in aqueous fluids and a pKₐ between about 3 and about 6, which result in a desired dissolution profile.

More particularly, the present invention relates to pharmaceutical compositions providing enhanced solubility parameters of bicalutamide prepared using hydrophilic excipient(s), other than polyvinylpyrollidones, which result in a desired dissolution profile.

Further, the present invention relates to stable pharmaceutical compositions providing enhanced solubility parameters of bicalutamide, which do not show changes in XRD pattern(s) upon storage.

XRD patterns described herein were obtained using a PAN analytical X-Ray Diffractometer (Model: X'Pert PRO^{™}) and X'Celerator detector with copper K-alpha radiation (1.541 A). In the XRD patterns, the horizontal axis is the 2θ angle in degrees and the vertical axis is intensity.

"Intimate mixture" in the present context of the invention is defined as a solid obtained by dissolving bicalutamide and a hydrophilic excipient in a suitable solvent or mixture of solvents and removing the solvents. While the invention is not to be constrained by any particular theory, the intimate mixtures can be considered as being in the nature of solid solutions.

In some instances, bicalutamide will be dissolved in a first solvent, hydrophilic excipient(s) will be dissolved in a different solvent that is miscible with the first solvent, and the solutions will be mixed. In other instances, solutions of bicalutamide and hydrophilic excipient(s) will be prepared separately using a solvent, and the solutions will be mixed. Provided the bicalutamide and hydrophilic excipient(s) are soluble in the same solvent, it can be advantageous to dissolve both components in a single portion of solvent.

In one aspect, the pharmaceutical compositions of the present invention having enhanced solubility parameters may comprise any amorphous or crystalline forms of bicalutamide, or mixtures of amorphous and crystalline forms of bicalutamide in varying ratios, optionally with other pharmaceutical excipients.

In an embodiment, the compositions of the present invention providing enhanced solubility parameters of bicalutamide may be intimate mixtures comprising bicalutamide and hydrophilic excipients.

Intimate mixtures can be prepared by techniques such as melt extrusion, spray-drying, freeze-drying, solvent controlled precipitation, fluidized bed processing, pH controlled precipitation, supercritical fluid technology and the like.

In an embodiment, pharmaceutical compositions providing enhanced solubility parameters of bicalutamide can be made by techniques such as, but not limited, to melt precipitation, homogeneous dispersion or solution of the active and hydrophilic excipients followed by removal of solvent by distillation, oven drying, tray drying, rotational drying (such as with the Buchi Rotavapor), fluidized bed drying, and the like.

In an embodiment, stable pharmaceutical compositions providing enhanced solubility parameters of bicalutamide can be prepared using one or more hydrophilic excipients to form an intimate mixture and these intimate mixtures are optionally mixed with suitable excipients and can be filled into capsules or compressed as tablets.

In one embodiment, pharmaceutical compositions of the present invention are prepared by a process comprising:
1. Dissolving a hydrophilic excipient and optionally a surfactant in a first solvent.
2. Dissolving bicalutamide in the first solvent or a different solvent that is miscible with the first solvent.
3. Mixing the solutions of step 1 and step 2 together to form a clear solution.
4. Removing solvent to provide a solid comprising hydrophilic excipient and bicalutamide or depositing solid onto pharmaceutically acceptable excipients while removing the solvent.
5. Preparing a pharmaceutical dosage form from solid or solid deposited onto excipients, optionally using one or more pharmaceutically acceptable excipients.

In an embodiment, pharmaceutical compositions of the present invention are prepared by a process comprising:
1. Dissolving a hydrophilic excipient and optionally a surfactant in water.
2. Dissolving bicalutamide in a suitable water-miscible solvent.
3. Mixing the solutions of step 1 and step 2 together to form a clear solution.
4. Granulating pharmaceutically acceptable excipients using the step 3 solution, and drying formed granules.
5. Mixing dried granules of step 4 with pharmaceutically acceptable excipients.
6. Compressing into tablets and optionally coating with a film coating polymer.

In various embodiments, weight ratios of bicalutamide to hydrophilic excipient range from about 2:1 to 1:2, or about 1.5:1 to 1:1.5, or about 1:1.

Suitable solvents for dissolving bicalutamide include, without limitation thereto: ketones such as acetone; alcohols such as ethanol, isopropyl alcohol, propanol, absolute alcohol, and methanol; other solvents such as tetrahydrofuran, chloroform, dichloromethane, toluene, polyethylene, 1,2-dichloroethane, and diethyl ether; and combinations of any two or more thereof.

Various hydrophilic pharmaceutically acceptable excipients that may be used with bicalutamide include but are not limited to: cellulose derivatives such as carboxymethyl cellulose, hydroxypropyl cellulose, hydroxyethyl cellulose, and hydroxypropyl methylcellulose or hypromellose (commercially available as Methocel, supplied by Dow Chemical Company). Various grades of Methocel^{®} with different viscosity grades available for example Methocel^{®} K 100 Premium LVEP, Methocel^{®} K4M premium, Methocel^{®} K15M premium, Methocel^{®} K100M *Premium,* Methocel^{®} *E4M Premium,* Methocel^{®} F50 *Premium,* Methocel^{®} E10M Premium CR, Methocel^{®} E3 Premium LV, Methocel^{®} E5 Premium LV, Methocel^{®} E6 Premium LV, Methocel^{®} E15 Premium LV, Methocel^{®} E50 Premium LV; potassium methacrylate/divinylbenzene copolymers; cyclodextrins; polyhydric alcohols; polyvinylpyrrolidone or povidone; and the like; and copolymers of the above polymers or mixtures of any two or more in various ratios as desired, for example combinations of methylcellulose and hydroxypropyl methyl cellulose (commercially available as Metolose^{®} supplied by Shin-Etsu Chemical in different grades such as Metolose^{®} *60SH,* Metolose^{®} 65SH, Metolose^{®} *90SH*. Some of these materials are not truly soluble in certain solvents such as water, but undergo swelling to form colloidal dispersions.

Various surfactants that may be used with bicalutamide include non-ionic, cationic, or anionic surface active agents, and natural surfactants. Useful non-ionic surface active agents include but are not limited to ethylene glycol stearates, propylene glycol stearates, diethylene glycol stearates, glycerol stearates, sorbitan esters (SPAN^{™}) and polyhydroxy ethylenically treated sorbitan esters (TWEEN^{™}), aliphatic alcohols and PEG ethers, phenol and PEG ethers. Useful cationic surfactants agents include but not limited to quaternary ammonium salts (e.g. cetyltrimethylammonium bromide) and amine salts (e.g. octadecylamine hydrochloride). Useful anionic surfactants include but are not limited to sodium stearate, potassium stearate, ammonium stearate, and calcium stearate; triethenolamine stearate, sodium lauryl sulphate, sodium dioctylsulphosuccinate, and sodium dodecylbenzenesulphonate. Natural surface active agents may also be used, such as for example phospholipids, e.g. diacylphosphatidyl glycerols, diaceylphosphatidyl cholines, and diaceylphosphatidic acids, the precursors and derivatives thereof.

In an embodiment, the starting bicalutamide for making pharmaceutical compositions providing enhanced solubility parameters of bicalutamide can either be crystalline, amorphous or mixtures thereof.

In an embodiment, pharmaceutical compositions providing enhanced solubility parameters of bicalutamide may be prepared in one or more steps. For example, pharmaceutical compositions providing enhanced solubility parameters of bicalutamide may be obtained by in situ formation, such as coprecipitation, of solid solutions of bicalutamide during preparation of such compositions. Alternatively, such solid solutions of bicalutamide may be prepared according to the above-mentioned, or other, processes, and further converted into suitable pharmaceutical compositions (finished dosage forms) by well-known techniques.

In the context of the present invention, during the production of finished dosage forms using the pharmaceutical compositions, one or more pharmaceutically acceptable excipients may optionally be used which include but are not limited to: diluents such as microcrystalline cellulose (MCC), including various grades sold as AVICEL^{™} by FMC BioPolymer, silicified MCC (e.g. PROSOLV^{™} HD 90), microfine cellulose, lactose, starch, pregelatinized starch, mannitol, sorbitol, dextrates, dextrin, maltodextrin, dextrose, calcium carbonate, calcium sulfate, dibasic calcium phosphate dihydrate, tribasic calcium phosphate, magnesium carbonate, magnesium oxide and the like; binders such as acacia, guar gum, alginic acid, dextrin, maltodextrin, methylcellulose, ethylcellulose, hydroxyethyl cellulose, hydroxypropyl cellulose (e.g. KLUCEL^{®}), hydroxypropyl methylcellulose (e.g. METHOCEL^{®}), carboxymethylcellulose sodium, povidone (various grades of KOLLIDON^{®}, PLASDONE^{®}), starch and the like; disintegrants such as carboxymethyl cellulose sodium (e.g. Ac-Di-Sol^{®}, PRIMELLOSE^{®}), crospovidone (e.g. Kollidon CL^{®}, Polyplasdone^{®}), polacrilin potassium, starch, pregelatinized starch, sodium starch glycolate (e.g. Explotab^{®}) and the like; surfactants including anionic surfactants such as chenodeoxycholic acid, 1-octanesulfonic acid sodium salt, sodium deoxycholate, glycodeoxycholic acid sodium salt, N-lauroylsarcosine sodium salt, lithium dodecyl sulfate, sodium cholate hydrate, sodium lauryl sulfate (SLS) and sodium dodecyl sulfate (SDS); cationic surfactants such as cetylpyridinium chloride monohydrate and hexadecyltrimethylammonium bromide; nonionic surfactants such as N-decanoyl-N-methylglucamine, octyl α-D-glucopyranoside, n-Dodecyl b-D-maltoside (DDM), polyoxyethylene sorbitan esters like polysorbates and the like; plasticizers such as acetyltributyl citrate, phosphate esters, phthalate esters, amides, mineral oils, fatty acids and esters, glycerin, triacetin or sugars, fatty alcohols, polyethylene glycol, ethers of polyethylene glycol, fatty alcohols such as cetostearyl alcohol, cetyl alcohol, stearyl alcohol, oleyl alcohol, myristyl alcohol and the like. Solvents that can be used in layering or coating are aqueous like water, or alcoholic like ethanol, isopropanolol, or hydro-alcoholic like a mixture of water with alcohol in any ratio, or organic like acetone, methylene chloride, dichloromethane and the like.

The lists above are not intended to be comprehensive, but provide representative examples of materials that can be used. Many other materials will be found useful in the invention, as is appreciated by those skilled in the art.

Pharmaceutical compositions may further include other excipients such as, but not limited to, pharmaceutically acceptable glidants, lubricants, opacifiers, colorants and other commonly used excipients.

In certain aspects, the pharmaceutical compositions of the present invention can be manufactured by dry or wet granulation. The granulate particles, optionally with suitable pharmaceutical excipients, may be compressed into tablets or filled into capsules.

In some embodiments, pharmaceutical compositions of the present invention providing enhanced solubility may have cores onto which bicalutamide or a bicalutamide composition is deposited. Non-limiting examples of such cores include water-soluble cores such as sugar spheres, lactose and the like; and water-insoluble cores such as microcrystalline cellulose, silicon dioxide, dibasic calcium phosphate, glass beads and the like.

The pharmaceutical compositions of the present invention can further be optionally film coated by techniques known to one skilled in the art such as powder coating, spray coating, dip coating, fluidized bed coating and the like.

Pharmaceutical compositions of bicalutamide as disclosed in context of the present invention are useful in the treatment of prostatic cancer.

The following examples are provided only to further illustrate certain specific aspects and embodiments of the invention in greater detail, and are not intended to limit the scope of the invention in any manner.

### EXAMPLE 1: Bicalutamide 50 mg tablets.

| **Ingredient** | **Grams/Batch** |
|---|---|
| Bicalutamide | 100 |
| Hypromellose, 3 cps * | 100 |
| Sodium lauryl sulphate | 40 |
| Acetone | 1000 |
| Water | 500 |
| Microcrystalline cellulose (AVICEL PH102) | 70 |
| Crospovidone ** | 30 |
| Lactose anhydrous (Pharmatose DCL21) ^{$} | 40 |
| Microcrystalline cellulose (AVICEL PH102) | 362 |
| Crospovidone ** | 40 |
| Magnesium stearate | 8 |

| | |
|---|---|
| * Hypromellose, 3 cps is manufactured by Dow Chemical Co. ** Crospovidone is manufactured by BASF. ^{$} Pharmatose DCL21 is manufactured by DMV-Fonterra. | |

### Manufacturing process:

1. Hypromellose and sodium lauryl sulphate were dissolved in water.
2. Bicalutamide was dissolved in acetone.
3. The solutions of step 1 and step 2 were mixed together.
4. Microcrystalline cellulose, crospovidone and lactose anhydrous were sifted through a ASTM # 30 mesh sieve and were granulated in a fluid bed granulator using step 3 solution at the following process parameters: inlet air temperature: 55-65 °C; inlet air blower rpm: 600-1200; atomization air pressure: 1.2-1.8 kg/cm².
5. The granules were sifted through a ASTM # 24 mesh sieve and were mixed with microcrystalline cellulose; crospovidone and magnesium stearate (sifted through a ASTM # 60 mesh sieve).
6. The lubricated blend was compressed into 2,000 tablets using 11 mm round punches and corresponding dies.

The tablets were subjected to a comparative *in vitro* dissolution study with the following parameters:
Method: Test 711 "Dissolution" in *United States Pharmacopeia 29*, United States Pharmacopeial Convention, Inc., Rockville, Maryland, 2005 ("USP"). Media: 1000 ml water with 0.5 % w/v sodium lauryl sulfate.
Apparatus: USP Type II apparatus (paddle).
Agitation: 50 rpm.
Reference: CASODEX^{™} 50 mg Tablets.

| **Time** **(minutes)** | **Cumulative % Drug Released** | |
|---|---|---|
| | **Reference** | **Example 1** |
| 10 | 65 | 57 |
| 20 | 89 | 72 |
| 30 | 94 | 81 |
| 60 | 97 | 91 |

### EXAMPLE 2: Bicalutamide 50 mg tablets

| **Ingredient** | **Grams/Batch** |
|---|---|
| Bicalutamide | 100 |
| Hypromellose, 5 cps * | 200 |
| Sodium lauryl sulphate | 40 |
| Acetone | 1000 |
| Water | 500 |
| Microcrystalline cellulose (AVICEL PH102) | 80 |
| Crospovidone | 30 |
| Lactose anhydrous (Pharmatose DCL21) ^{$} | 40 |
| Microcrystalline cellulose (PH102) | 356 |
| Crospovidone | 88 |
| Dicalcium phosphate | 452 |
| Lactose anhydrous (Pharmatose DCL21) ^{$} | 198 |
| Magnesium stearate | 16 |

| | |
|---|---|
| * Hypromellose, 5 cps is manufactured by Dow Chemical Co. ^{$} Pharmatose DCL21 is manufactured by DMV-Fonterra. | |

### Manufacturing process:

1. Hypromellose and sodium lauryl sulfate were dissolved in water.
2. Bicalutamide was dissolved in acetone.
3. The solutions of step 1 and step 2 were mixed together
4. Microcrystalline cellulose, crospovidone and lactose anhydrous were sifted through a ASTM # 30 mesh sieve and were granulated in a fluid bed granulator using step 3 solution at the following process parameters: inlet air temperature: 55-65 °C; inlet air blower rpm: 600-1200; atomization air pressure: 1.2-1.8 kg/cm².
5. The granules were sifted through a ASTM # 24 mesh sieve and were mixed with microcrystalline cellulose, crospovidone and magnesium stearate (sifted through a ASTM # 60 mesh sieve).
6. The lubricated blend was compressed into 2,000 tablets using 11 mm round punches and corresponding dies.

The tablets were subjected to a comparative *in vitro* dissolution study with the following parameters.
Media: 1000 ml water with 0.5 % w/v sodium lauryl sulfate.
Apparatus: USP Type II apparatus (paddle).
Agitation: 50 rpm.
Reference: CASODEX^{™} 50 mg tablets.

| **Time** **(minutes)** | **Cumulative % Drug Released** | |
|---|---|---|
| | **Reference** | **Example 2** |
| 10 | 65 | 70 |
| 20 | 89 | 86 |
| 30 | 94 | 92 |
| 60 | 97 | 98 |

### EXAMPLE 3: Bicalutamide 50 mg tablets.

| **Ingredient** | **Grams/Batch** |
|---|---|
| Bicalutamide | 100 |
| Hypromellose, 3 cps * | 100 |
| Sodium lauryl sulphate | 20 |
| Acetone | 1000 |
| Water | 50 |
| Microcrystalline cellulose (PH 102) | 220 |
| Sodium starch glycolate | 20 |
| Lactose anhydrous (Pharmatose DCL21)^{$} | 150 |
| Microcrystalline cellulose (AVICEL PH102) | 44 |
| Sodium starch glycolate | 36 |
| Magnesium stearate | 10 |

| | |
|---|---|
| * Hypromellose, 3 cps is manufactured by Dow Chemical Co. ^{$} Pharmatose DCL21 is manufactured by DMV-Fonterra. | |

### Manufacturing process:

1. Hypromellose and sodium lauryl sulfate were dissolved in water.
2. Bicalutamide was dissolved in acetone.
3. The solution of step 1 and step 2 were mixed together.
4. Microcrystalline cellulose, sodium starch glycolate and lactose anhydrous were sifted through a #30 mesh sieve and were granulated in fluid bed granulator using step 3 solution at the following process parameters: inlet air temperature: 55-65 °C; inlet air blower rpm: 600-1200; atomization air pressure: 1.2-1.8 kg/cm².
5. The granules were sifted through a ASTM # 24 mesh sieve and were mixed with microcrystalline cellulose, sodium starch glycolate and ASTM # 60 mesh sieve-passed magnesium stearate.
6. The lubricated blend was compressed into 2,000 tablets.

Examples 4 and 5 study the effect of the grade of hypromellose in the formulation.

### EXAMPLE 4: Bicalutamide solid solution using hypromellose (3 cps)

Manufacturing procedure:
1. 5 g of hypromellose (3 cps) was dissolved in 100 g of water.
2. 5 g of bicalutamide was dissolved in 100 g of acetone.
3. The solutions of steps 1 and step 2 were mixed together and spray dried in a mini spray drier (Buchi) at an inlet air temperature of 80 °C.

### EXAMPLE 5: Bicalutamide solid solution using hypromellose (5 cps)

Manufacturing procedure:
1. 10 g of hypromellose (5 cps) was dissolved in 100 g of water.
2. 5 g of bicalutamide was dissolved in 100 g of acetone.
3. The solutions of steps 1 and step 2 were mixed together and spray dried in a mini spray drier (Buchi) at an inlet air temperature of 80 °C.

The intimate mixtures formed by spray drying in Examples 4 and 5 were subjected to an *in vitro* dissolution study with the following parameters:
Media: 1000 ml, 1 % w/v sodium lauryl sulfate.
Apparatus: USP Type II apparatus (paddle).
Agitation: 50 rpm.

| **Time** **(minutes)** | **Cumulative % Drug Released** | |
|---|---|---|
| | **Example 4 (n=3)** | **Example 5 (n=6)** |
| 0 | 0 | 0 |
| 30 | 92 | 91 |
| 60 | 93 | 98 |

### EXAMPLE 6: Bicalutamide 50 mg tablets.

| **Ingredient** | **Kg/Batch** |
|---|---|
| Bicalutamide | 6.5 |
| Hypromellose, 3 cps | 6.5 |
| Sodium lauryl sulfate | 1.3 |
| Acetone | 39 |
| Water | 13 |
| Microcrystalline cellulose (AVICEL PH101) | 2.6 |
| Lactose monohydrate (impalpable) | 5.2 |
| Crospovidone | 4.55 |
| Microcrystalline cellulose (Avicel PH 102) | 24.83 |
| Magnesium stearate | 0.52 |
| Opadry^{™} White OY-58900 | 1.56 |
| Isopropyl alcohol | 10.4 |
| Water | 4.29 |

| | |
|---|---|
| Opadry^{™} White OY-58900 comprises hypromellose 5 cps, polyethylene glycol, and titanium dioxide, and is supplied by Colorcon. AVICEL PH101 and Avicel PH102 are supplied by FMC Bio Polymer. | |

Manufacturing procedure:
1. Sodium lauryl sulfate was dispersed in water under stirring to form a uniform dispersion.
2. Bicalutamide was dissolved in acetone, hypromellose 3 cps was added and the mixture stirred for 5 minutes to form a dispersion.
3. The dispersion from step 1 was added to drug and hypromellose dispersion of step 2 and stirred until a clear to slightly turbid solution was obtained.
4. Avicel PH 101, lactose monohydrate, and crospovidone were sifted through a ASTM 40 mesh sieve and placed into fluid bed granulator.
5. Above step 5 materials were granulated using step 3 solution using a top spray assembly.
6. The granules were dried at an inlet air temperature of 60 °C ± 5 °C for 1 hour until loss on drying is 0.5-3 % w/w.
7. The granules were sifted through a ASTM 40 mesh sieve.
8. Avicel PH 102, crospovidone were sifted through a ASTM 40 mesh sieve, and magnesium stearate was sifted through a ASTM 60 mesh sieve.
9. Dried granules and sifted Avicel PH 102 and crospovidone from step 8 were placed into a double cone blender and blended for 20 minutes.
10. Sifted magnesium stearate from step 8 was added to step 9 and blended for 5 minutes.
11. The lubricated blend from step 10 was compressed into 1,300 tablets.
12. Opadry White OY-58900 was dispersed in isopropyl alcohol, then water was added and the mixture stirred for about 30 minutes.
13. The tablets from step 11 were coated using Opadry dispersion prepared in step 12.
14. Coated tablets were placed into closed HDPE (high-density polyethylene) bottles and stored at 40°C and 75% relative humidity, in a stability test.

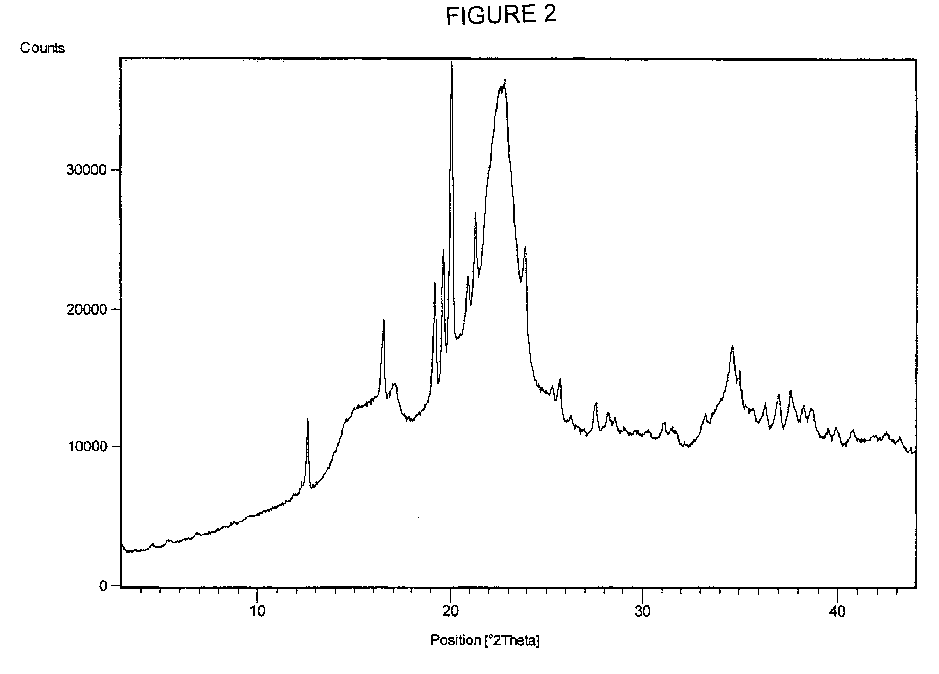

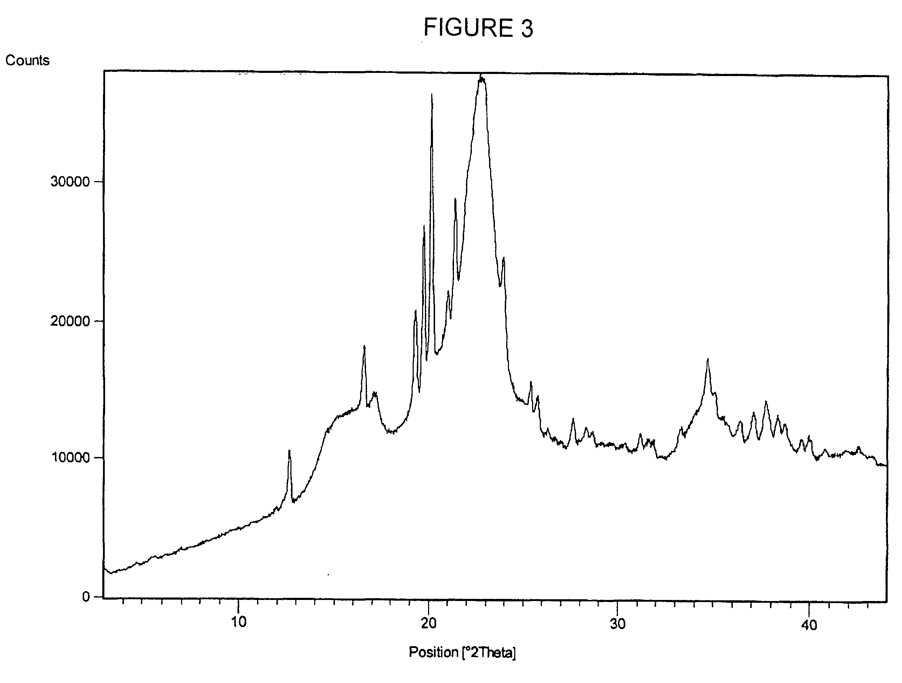

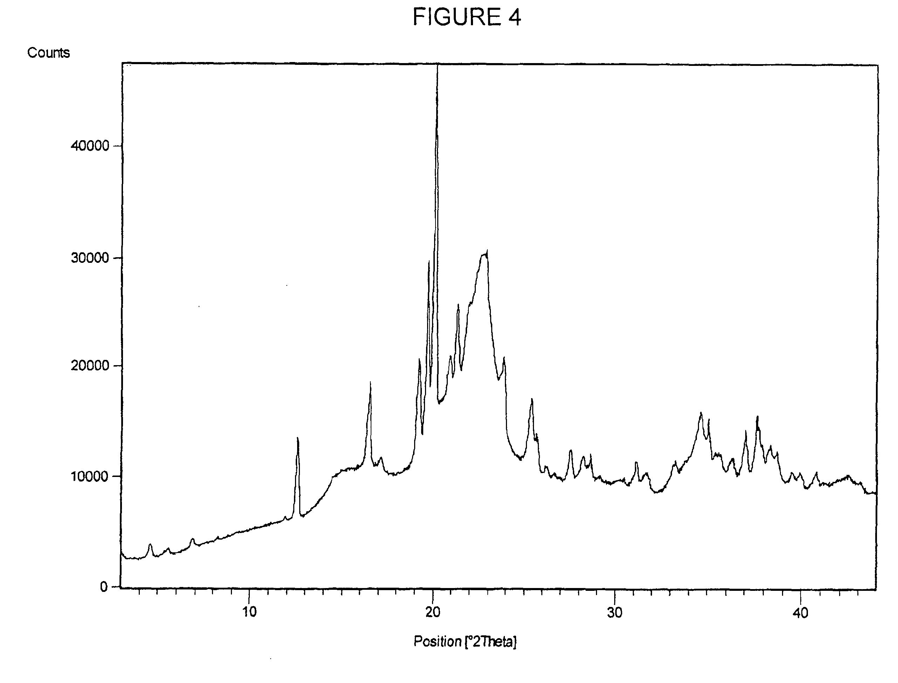

Initial and stability samples were analyzed by X-ray powder diffraction. XRD patterns for initial tablets, tablets after storage for 3 months at 40°C and 75% RH, and placebo tablets (prepared as above, but omitting the bicalutamide) are shown in Figures 2, 3, and 4, respectively. A comparison of the XRD patterns for initial tablets and placebo revealed that initial tablets have substantially no peaks other than placebo peaks. Also, comparison of XRD patterns for initial tablets and tablets after storage at 40°C and 75% RH determined that substantially no peaks other than placebo peaks were present.

### EXAMPLE 7: Bicalutamide 50 mg tablets

| **Ingredient** | **Grams/Batch** |
|---|---|
| Bicalutamide | 100 |
| Hypromellose, 3 cps | 100 |
| Sodium lauryl sulfate | 20 |
| Acetone | 600 |
| Water | 200 |
| Microcrystalline cellulose (Avicel PH 101) | 40 |
| Crospovidone | 30 |
| Lactose mnohydrate | 80 |
| Microcrystalline cellulose (Avicel PH 102) | 390 |
| Crospovidone | 32 |
| Magnesium stearate | 8 |

Manufacturing process: similar to that of Example 6.

### EXAMPLE 8: Bicalutamide 50 mg tablets

| **Ingredient** | **Grams/Batch** |
|---|---|
| Bicalutamide | 100 |
| Hypromellose, 3 cps | 80 |
| Sodium lauryl sulfate | 20 |
| Acetone | 600 |
| Water | 200 |
| Microcrystalline cellulose (Avicel PH 101) | 40 |
| Crospovidone | 30 |
| Lactose monohydrate | 80 |
| Microcrystalline cellulose (Avicel PH 102) | 402 |
| Crospovidone | 40 |
| Magnesium stearate | 8 |
| Opadry™ White (OY-58900) | 54 |
| Isopropyl alcohol | 340 |
| Water | 146 |

Manufacturing process: similar to that of Example 6.

### EXAMPLE 9: Bicalutamide 50 mg tablets

| **Ingredient** | **Grams/Batch** |
|---|---|
| Bicalutamide | 100 |
| Hypromellose, 3 cps | 100 |
| Sodium lauryl sulfate | 20 |
| Acetone | 600 |
| Water | 200 |
| Microcrystalline cellulose (Avicel PH 101) | 40 |
| Crospovidone | 30 |
| Lactose monohydrate | 80 |
| Microcrystalline cellulose (Avicel PH 101) | 382 |
| Crospovidone | 40 |
| Magnesium stearate | 8 |
| Opadry^{™} White (OY-58900) | 54 |
| Isopropyl alcohol | 340 |
| Water | 146 |

Manufacturing process: similar to that of Example 6.

### EXAMPLE 10: Bicalutamide 50 mg tablets

| **Ingredient** | **Grams/Batch** |
|---|---|
| Bicalutamide | 100 |
| Hypromellose, 3 cps | 100 |
| Sodium lauryl sulfate | 60 |
| Acetone | 1170 |
| Water | 700 |
| Microcrystalline cellulose (Avicel PH 102) | 80 |
| Crospovidone | 30 |
| Lactose anhydrous (DCL 21) | 40 |
| Microcrystalline cellulose (Avicel PH 102) | 342 |
| Crospovidone | 40 |
| Magnesium stearate | 8 |
| Opadry^{™} White (OY-58900) | 27 |
| Isopropyl alcohol | 170 |
| Water | 73 |

Manufacturing process: similar to that of Example 6.

### EXAMPLE 11: Comparative dissolution profiles of the tablets prepared in Examples 7, 8, 9 and 10.

Media: Water with 1% sodium lauryl sulfate.
Volume: 1000 ml.
Apparatus: USP Type II apparatus (paddle).
Agitation: 75 rpm.
Reference: CASODEX^{™} 50 mg tablets.

| **Time** **(minutes)** | **Cumulative % Drug Released** | | | | |
|---|---|---|---|---|---|
| | **Example 7** | **Example 8** | **Example 9** | **Example 10** | **Reference** |
| 15 | 84 | 75 | 84 | 28 | 89 |
| 30 | 97 | 90 | 97 | 61 | 97 |
| 45 | 101 | 96 | 102 | 78 | 97 |
| 60 | 103 | 98 | 104 | 93 | 98 |
| 75 | 103 | 100 | 105 | 99 | 97 |

## Claims

1. An intimate mixture comprising bicalutamide and a hydropic excipient that is not an enteric polymer or a polyvinylpyrrolidone.

2. The intimate mixture of claim 1, further comprising a surfactant.

3. The intimate mixture of claim 1, further comprising sodium lauryl sulfate.

4. The intimate mixture of any of claims 1-3, wherein a hydrophilic excipient comprises a hypromellose.

5. The intimate mixture of any of claims 1-4, prepared by a process comprising preparing a solution comprising bicalutamide and a hydrophilic excipient, and removing solvent.

6. The intimate mixture of claim 5, wherein a solution comprises bicalutamide and acetone.

7. The intimate mixture of any of claims 1-6, wherein the hydrophilic excipient does not have pH-dependent solubility in aqueous fluids.

8. The intimate mixture of any of claims 1-6, wherein the hydrophilic excipient has a pKₐ that is not between about 3 and about 6.

9. A pharmaceutical composition comprising the intimate mixture of any of claims 1-8 and one or more pharmaceutically acceptable excipients.

10. The pharmaceutical composition of claim 9, which is in the form of a tablet.
